# EUROPEAN PATENT APPLICATION

(11) **EP 2 749 504 A1**
(43) Date of publication of application: **02.07.2014**
(21) Application number: 12853888.1
(22) Date of filing: 21.11.2012
(51) Int. Cl.: B65D 27/16, B65D 75/30, B65D 33/20, A61J 1/03

(54) **DEVICE AND METHOD FOR STORING AND PROVIDING DOSES OF HEALTH PRODUCTS**

(30) Priority: 28.11.2011 ES 201131919
(71) Applicant: Valgraf Europa S.L., 46960 Aldaya (Valencia) (ES)
(72) Inventor: OCHOA MARIN, Antonio, E-46960 Aldaya (Valencia) (ES)
(74) Representative: Ungria López, Javier
(86) International application number: PCT/ES2012/070808
(87) International publication number: WO 2013/079749

(57) **Abstract**

Device and method for storing and providing doses of health products. The device comprises at least two pouches (1), corresponding to the doses for a patient and connected laterally by means of a perforated line (7). Each pouch (1) comprises a first sheet (2) that is connected to the sides of a second sheet (3), one of the sides of the first (2) and second sheet (3) being unconnected in order to define an opening providing access to the pouch (1). Furthermore, the pouches comprise an adhesive strip (4) covered by a disposable protector (5), provided in the zone of the open side of one of the sheets, for closing the pouch (1). The pouches (1) form a band or sheet divided into a plurality of pouches (1). The band or sheet forms a reel. The invention relates, moreover, to a method that facilitates storing and providing doses, for a patient, in the form of pouches connected by perforated lines.

## Description

### OBJECT OF THE INVENTION

The present invention, as expressed in the heading of this specification, relates to a device and a method making it possible to store and provide doses of health products, the object of which is to optimize the packaging and dosing of health products, for example drugs, while making it possible as well to temporarily store said drugs neatly and aseptically, in order to administer them to any given patient.

The invention may also be employed to temporarily, neatly and aseptically store prostheses, or any other product belonging to the patient, during surgery or medical testing.

### BACKGROUND OF THE INVENTION

In the state of the art, both dosages of drugs and prostheses of hospitalized patients are held for safekeeping in trays or individual containers.

In the case of providing doses of drugs to hospitalized patients, small cups or trays are used, wherein, in accordance with the medical prescription, pharmacists prepare the doses, which the nurses then distribute to the rooms.

This procedure sometimes gives rise to serious problems such as loss of drugs, incorrect dosages, low sterility, etc., with the severe health consequences that accompany such problems.

### DESCRIPTION OF THE INVENTION

To meet the objectives and overcome the drawbacks outlined above, the invention provides a new device making it possible to store a health product and later provide a patient with doses thereof, to which end it is characterized in that it comprises at least two pouches connected to one another on one of their sides by means of a perforated line. Each one of the pouches comprises a first sheet that is superimposed upon and is connected to the two sides of a second sheet, such that one of said sides of the first and second sheets are unconnected, thus defining an opening providing access to the inside of the pouch. Furthermore, each one of the pouches comprises an adhesive strip that is covered by a disposable protector, in such a way that both are provided in the zone of the opening side of either the first sheet or the second sheet, making it possible to seal the pouch by affixing the sheet without the adhesive strip onto the sheet provided with said adhesive strip, after removing the disposable protector. The disposable protector and the adhesive strip of the at least two pouches are made up of a continuous band, and delimited by means of the perforated line.

In one embodiment of the invention, the adhesive strip and its corresponding disposable protector are located on the inner surface of either the first sheet or the second sheet. This arrangement makes it possible for the sheet without the adhesive strip to be superimposed upon said adhesive strip, in such a way that after removing the disposable protector, the sheet without the adhesive strip can be easily affixed onto said adhesive strip. In this arrangement, it is also possible for the sheet with the adhesive strip on its inner surface to protrude with respect to the sheet without said adhesive strip, such that the adhesive strip together with the disposable protector are located on the protruding portion, so as to seal the pouch following removal of the disposable protector and folding the protruding portion over the sheet without the adhesive strip.

In another embodiment of the invention, it is provided that the adhesive strip and the corresponding disposable protector be provided on the outer surface of the first or second sheet near its open side, such that the sheet without the adhesive strip protrudes on its open side with respect to the sheet with the adhesive strip, such that this arrangement makes it possible to seal the pouch following removal of the disposable protector and folding the protruding portion of the sheet without the adhesive strip over the corresponding sheet with the adhesive strip.

The invention provides that the adhesive strip may be either a permanently-sealing adhesive strip, or a resealable adhesive strip, i.e. it may be opened, such that in the first case the pouch must be torn open to gain access to its contents, whereas in the second case the pouch may be reused.

In the case in which the seal is permanent, one of the sheets, the first, the second, or both, comprises a perforated line for easy opening.

The invention provides that the first sheet be made of a printable material, so that the information corresponding to the patient may be printed thereupon, such as the room where the patient is located, the dose of the stored product, the product that has been stored, and/or the time at which the stored dose should be administered.

The preferred embodiment of the invention provides that the sheet of printable material be paper, though it may obviously be made of any other material.

With respect to the second sheet, it is worth pointing out that it is made of a transparent material in the preferred embodiment of the invention, though it may also be made of any other material. The transparent material of the second sheet is an oxo-degradable plastic.

The invention provides that the device of the invention comprises a plurality of pouches connected laterally by means of perforated lines, such that the device forms a band-like arrangement in which the pouches are connected one after another, wherein said band may form a reel, from which the various pouches or groups of pouches may be separated by tearing along the perforated lines. This arrangement makes it possible to separate some pouches from others, in groups or individually.

In a similar way, the pouches may be also connected to one another by means of perforated lines forming a sheet, such that a plurality of sheets connected to one another by perforated lines may also be provided, forming a band or a reel.

Furthermore, the invention relates to a method making it possible to store and provide doses of health products, which comprises a step that requires obtaining at least two pouches, corresponding to the doses required by a patient, and connected on one of their sides by means of a perforated line, in such a way that said pouches include sealing means. Next, a pre-established dose of a health product previously prescribed to the patient is placed inside each of the pouches, such that the pouches containing the doses of the health product may be sealed, said doses thereby remaining in storage inside the pouch until they can be administered to the patient. In this way, at least two doses can be grouped together, as when they are to be administered to the patient on the same day, for example.

To administer each one of the doses to the patient, the perforated line is torn in order to separate a pouch to be subsequently administered to the patient. As mentioned previously, for the device of the invention, the adhesive strip may be either permanently-sealing, or resealable, such that in the first case the pouch must be torn open, as mentioned previously, whereas in the resealable case the pouch may be reused.

Furthermore, the method of the invention comprises a step, prior to the step in which the pouches are sealed, wherein information is printed on said pouches. It has been provided that said information to be printed may be information pertaining to the patient, to the room where the patient is located, to the dose of the stored product, to the product that is being stored, to the times or periods for administering the dose stored in each pouch, and any combination thereof. This step is carried out by printing said information on the first sheet of printable material. The printing step may be carried out by hand, or by means of a printer or any other conventional means known in the state of the art.

The device and method described herein optimize the packaging and provision of doses of health products, such as drugs.

For the purpose of helping to make this specification more readily understandable, a set of drawings constituting an integral part of the same has been included below, wherein by way of illustration and not limitation the object of the invention has been represented.

### BRIEF OUTLINE OF THE DRAWINGS

**Figure 1****.-** Shows a view of a possible exemplary embodiment of the device of the invention, wherein it comprises a plurality of pouches connected one after another in a series of pouches, by means of perforated lines.
**Figure 2****.-** Shows a side view of the sealing zone of each one of the pouches in accordance with the embodiment in Figure 1.
**Figure 3****.-** Shows another exemplary embodiment of the sealing zone of the pouches.
**Figure 4****.-** Shows another possible way of connecting the pouches, forming a sheet-like arrangement.

### DESCRIPTION OF THE PREFERRED EMBODIMENT

What follows is a description of the invention based on the figures described above.

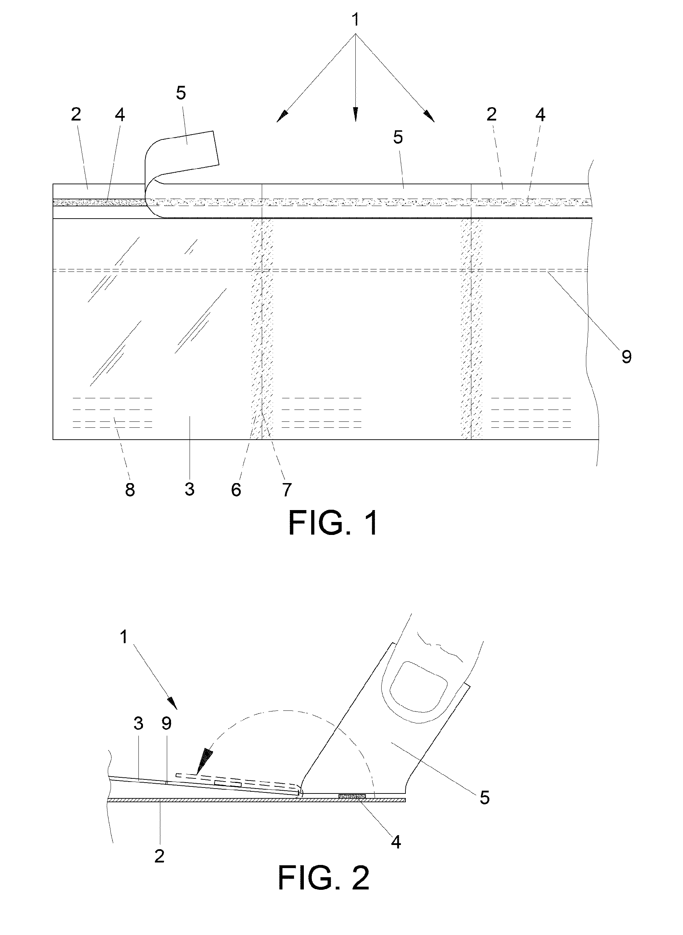

Figure 1 shows an exemplary embodiment in which the device of the invention is constituted by a plurality of pouches 1 connected on one of their sides by means of a perforated line 7.

The pouches are constituted by a first sheet 2, upon which there is a second sheet 3 superimposed that is connected to the sides of the first sheet 2, except for one of its sides, thus defining an opening providing access to the inside of the pouches 1. In the example in Figure 1, the first sheet 2 and the second sheet 3 take the form of a continuous band on which an adhesive is provided on the sides of the contour of said sheet, along with parallel and evenly-spaced transversal lines of adhesive 6, which define the different pouches 1. A perforated line 7 is made along the transversal lines of adhesive 6, so that by tearing along said perforated line 7 the different pouches 1 forming the aforementioned continuous band may be separated.

Furthermore, in the exemplary embodiment in Figure 1, the first sheet 2 is longer than the second sheet 3, such that on the inner surface of the first sheet 2 and on the protruding section, there is an adhesive strip 4 covered by a disposable protector 5. Said adhesive strip and disposable protector 5 of each pouch form a continuous band, and are delimited by means of the aforementioned perforated line 7.

Based on the above description, it is easy to see how a group of pouches 1 may be separated, for example, a group corresponding to the number of doses that a patient will need throughout the day, by tearing along the corresponding perforated lines. Next, the pouches are filled with the dose of a health product prescribed to a patient, and thereupon the disposable protector 5 of each pouch is removed, and the area of the first sheet 2 bearing the adhesive strip 4 is folded over the outer surface of the second sheet 3, in such a way that each pouch 1 is thus sealed, storing each of the doses of health product required by the patient, for example over the course of a day, until they can be administered to the patient.

In the preferred embodiment of the invention, the first sheet 2 is made of a printable material, such as paper, and the second sheet 3 is made of a transparent plastic material, through which the product stored inside the pouch can be seen. In the preferred embodiment, the plastic is oxo-degradable.

On the outer surface of the first sheet 2, an area 8 is provided for printing various sorts of information pertaining to the patient, to the room where the patient is located, to the dose of the stored product, to the product itself, and to the frequency and/or times for administering the dose of health product contained in each pouch 1 to the patient.

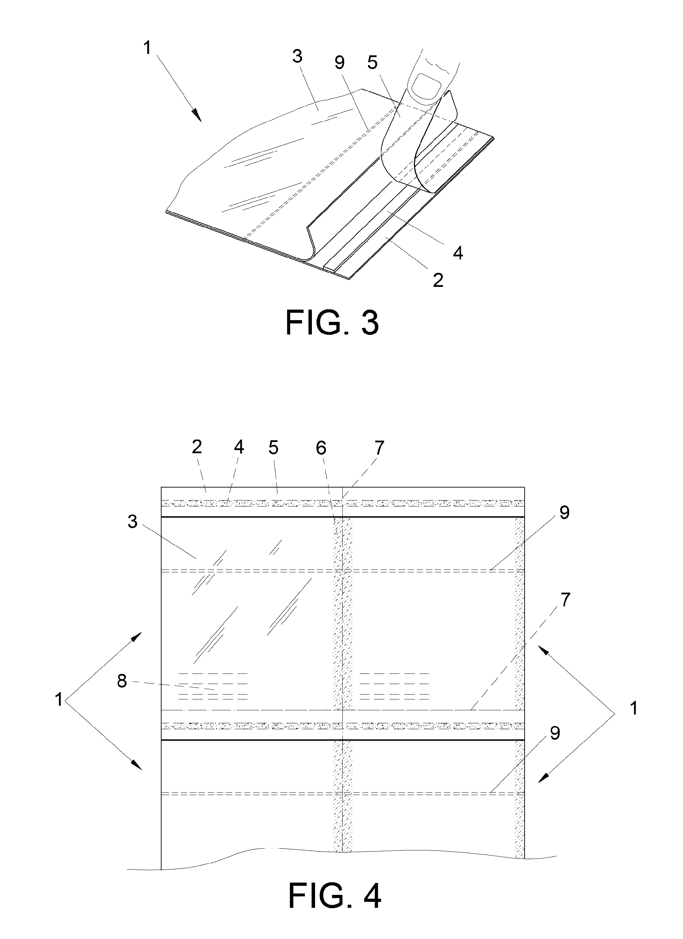

Figure 3 shows another possible exemplary embodiment in which the first sheet 2 has the same length as the second sheet 3, such that by removing the disposable protector 5, the adhesive strip 4 can be affixed to the inner surface of the second sheet 3, sealing the pouch 1, as shown in Figure 3.

Figure 4 shows another possible embodiment which provides a plurality of pouches 1 connected to one another laterally to form a sheet.

In any of the aforementioned cases, the pouches may be rolled into a reel, so as to easily obtain and separate the different pouches 1.

In Figure 2, the second sheet 3 is shown as being separated from the first sheet 2 because there is a health product inside the pouch.

The invention also relates to a method making it possible to store and provide doses of health products, comprising a step wherein the pouches must be separated as a group, at least two units connected on one of their sides by means of a perforated line. Next, a pre-established dose of a health product previously prescribed to the patient is placed inside each of the pouches, whereby the pouches containing the doses of the health product may thereupon be sealed, said doses then remaining in storage inside the pouch until they can be administered to the patient. In this way, at least two doses that are to be administered to the patient on the same day, for example, can be grouped together.

To administer each one of the doses to the patient, the perforated line 7 is torn in order to separate a pouch 1 to be subsequently administered to the patient. As mentioned previously, for the device of the invention, the adhesive strip 4 may be either permanently-sealing, or resealable, such that in the first case the opening of the pouch, as mentioned previously, must be torn open, whereas in the resealable case the pouch may be reused.

In the case in which the seal is permanent, the invention provides that one of the sheets, 2, 3 or both, may include a perforated line 9 so as to easily open the pouch by tearing along said perforated line 9.

Furthermore, the method of the invention comprises a step, prior to the step in which the pouches are sealed, wherein information may be printed on said pouches, as has already been described.

## Claims

1. **Device for storing and providing doses of health products, characterized in that** it comprises at least two pouches (1) connected on one of their sides by means of a perforated line (7); each pouch (1) in turn comprising:
- a first sheet (2) that is superimposed upon and is connected to the sides of
- a second sheet (3); wherein one of the sides of the first (2) and second sheets (3) of the at least two pouches is unconnected, thus defining an opening providing access to the pouch (1);
- an adhesive strip (4) covered by a disposable protector (5), provided in the zone of the open side of one sheet chosen between the first (2) and second sheet (3), for sealing each pouch (1), wherein the disposable protector and the adhesive strip of the at least two pouches are constituted by a continuous band and delimited by means of the perforated line (7).

2. **Device for storing and providing doses of health products,** according to claim 1, **characterized in that** the adhesive strip (4) and the disposable protector (5) are located on the inner surface of one sheet chosen between the first (2) and second sheet (3).

3. **Device for storing and providing doses of health products,** according to claim 2, **characterized in that** the sheet with the adhesive strip (4) and disposable protector (5) on its inner surface is chosen between a sheet superimposed upon the sheet without said adhesive strip (4), such that the pouch (1) is sealed following removal of the disposable protector (5); and a sheet that protrudes with respect to the sheet without said adhesive strip (4), wherein the adhesive strip (4) is located on the inner surface of the protruding portion, such that the pouch (1) is sealed following removal of the disposable protector (5) and folding the protruding portion of the sheet with the adhesive strip (4) over the sheet without the adhesive strip (4).

4. **Device for storing and providing doses of health products,** according to claim 1, **characterized in that** the adhesive strip (4) and the disposable protector (5) are located on the outer surface and near to the open side of the sheet chosen between the first (2) and second sheet (3); while the open side of the sheet without the adhesive strip (4) protrudes with respect to the sheet with the adhesive strip (4), such that the pouch (1) is sealed following the removal of the disposable protector (5) and folding the protruding portion of the sheet without the adhesive strip (4) over said sheet comprising said adhesive strip (4).

5. **Device for storing and providing doses of health products,** according to claim 1, **characterized in that** the adhesive strip (4) is chosen between a permanently-sealing adhesive strip and a resealable adhesive strip.

6. **Device for storing and providing doses of health products,** according to claim 1, **characterized in that** the first sheet (2) is made of a printable material.

7. **Device for storing and providing doses of health products,** according to claim 6, **characterized in that** the first sheet (2) is made of paper.

8. **Device for storing and providing doses of health products,** according to claim 1, **characterized in that** the second sheet (3) is made of a transparent material.

9. **Device for storing and providing doses of health products,** according to claim 8, **characterized in that** the transparent material of the second sheet (3) is an oxo-degradable plastic.

10. **Device for storing and providing doses of health products,** according to the preceding claims, **characterized in that** it comprises a plurality of pouches (1) connected laterally by means of perforated lines (7), forming an arrangement chosen between a band, a reel, a sheet divided into a plurality of pouches by means of perforated lines (7), and any combination thereof.

11. **Device for storing and providing doses of health products,** according to claim 5, **characterized in that** a sheet chosen between the first sheet, second sheet, and a combination of both, comprises a perforated line (9) for tearing open the pouch (1) when it has a permanent seal.

12. **Method for storing and providing doses of health products, characterized in that** it comprises the following steps:
- obtaining at least two pouches (1), corresponding to the doses required by a patient, and connected on one of their sides by means of a perforated line (7) and including sealing means;
- placing a pre-established dose of a health product inside the at least two pouches (1);
- sealing the pouches (1) containing the doses of health product, in order to store said doses of health product;
- tearing the perforated line in order to separate a pouch and to be subsequently administered;
- opening the separated pouch to administer the dose of health product to the patient;
- opening the second separated pouch to administer the dose of health product to the patient when required.

13. **Method for storing and providing doses of health products,** according to claim 12, **characterized in that**, prior to the step wherein the pouch containing the health product is sealed, the method comprises a step wherein information is printed on the separated pouch (1), chosen between information pertaining to the patient, to the room where the patient is located, to the dose of the stored product, to the stored product, to the times for administering the stored dose, and any combination thereof.

14. **Method for storing and providing doses of health products,** according to claim 13, **characterized in that** the step wherein the pouch (1) containing the dose of health product is opened, comprises a step chosen between a step wherein the pouch (1) is torn, if the pouch comprises permanent sealing means; and a step wherein the pouch (1) is opened, if said pouch comprises resealable sealing means.
